# EUROPEAN PATENT SPECIFICATION

(11) **EP 3 232 917 B1**
(45) Date of publication and mention of the grant of the patent: **18.09.2019**
(21) Application number: 15869378.8
(22) Date of filing: 17.12.2015
(51) Int. Cl.: A61B 5/02, A61B 5/0402, A61B 5/0476, A61B 5/0488

(54) **APPARATUS FOR THE ASSESSMENT OF THE LEVEL OF PAIN AND NOCICEPTION DURING GENERAL ANESTHESIA USING ELECTROENCEPHALOGRAM, PLETHYSMOGRAPHIC IMPEDANCE CARDIOGRAPHY, HEART RATE VARIABILITY AND THE CONCENTRATION OR BIOPHASE OF THE ANALGESICS**
VORRICHTUNG ZUR BEURTEILUNG DES NIVEAUS VON SCHMERZ UND NOZIZEPTION WÄHREND EINER ALLGEMEINEN ANÄSTHESIE UNTER VERWENDUNG VON EINES ELEKTROENZEPHALOGRAMMS, PLETHYSMOGRAPHISCHER IMPEDANZKARDIOGRAPHIE, HERZFREQUENZVARIABILITÄT UND DER KONZENTRATION ODER BIOPHASE DER ANALGETIKA
APPAREIL POUR L'ÉVALUATION DU NIVEAU DE DOULEUR ET DE NOCICEPTION AU COURS D'UNE ANESTHÉSIE GÉNÉRALE À L'AIDE D'UN ÉLECTROENCÉPHALOGRAMME, D'UNE CARDIOGRAPHIE D'IMPÉDANCE PLÉTHYSMOGRAPHIQUE, DE LA VARIABILITÉ DE LA FRÉQUENCE CARDIAQUE ET DE LA CONCENTRATION OU BIOPHASE DES ANALGÉSIQUES

(30) Priority: 18.12.2014 DK 201400742
(43) Date of publication of application: 25.10.2017
(73) Proprietor: QUANTIUM MEDICAL S.L., 08302 Mataró, Barcelona (ES); Hospital Clínic de Barcelona, 08036 Barcelona (ES)
(72) Inventor: JENSEN, Erik Weber, 08395 Sant Pol de Mar Barcelona (ES); GAMBÚS, Pedro Luis, 08008 Barcelona (ES)
(74) Representative: Kusche, Robert
(86) International application number: PCT/DK2015/000052
(87) International publication number: WO 2016/095918

(56) References cited:
- WO-A1-02/03861
- WO-A1-2010/134068
- WO-A1-2014/091291
- US-A- 6 042 548
- US-A1- 2011 301 435
- US-A1- 2012 041 279
- US-A1- 2013 211 208

## Description

### BACKGROUND OF THE INVENTION

### Introduction to anesthesia

Anesthesia has been defined as a drug induced state where the patient has lost consciousness, loss of sensation of pain or any other stimuli, i.e. analgesia, and amnesia of all the procedure. Furthermore the patient may be paralyzed as well. (Prys-Roberts C: Anesthesia: A practical or impossible construct? Br J Anaesth 1987; 59:1341-2). To obtain these objectives, the anesthesiologist can use different classes of drugs, mostly hypnotics and analgesics. This allow the patients to undergo surgery and other procedures without the distress and pain they would otherwise experience.

Continuously, the brain receives a vast amount of stimuli. However, the subject is only processing a small amount of it. When administering enough doses of hypnotics, the following loss of consciousness make that the patient does not perceive the stimuli, but the neurovegetative and somatic responses are not necessarily abolished. When administering enough doses of analgesics they block the nociceptive stimuli and prevent the neurovegetative and somatic responses. However they do not always produce a loss of consciousness and amnesia.

One of the objectives of modern anesthesia is to ensure adequate level of consciousness to prevent awareness without inadvertently overloading the patients with anesthetics which might cause increased postoperative complications. The overall incidence of intraoperative awareness with recall is about 0.2-2%, but it may be much higher in certain high risk patients like multiple trauma, caesarean section, cardiac surgery and hemodynamically unstable patients. Sometimes the patients could remember noises or even voices. In other cases the patients cannot move and advice to the anesthesiologist that they are awake or in the worst case in pain during the surgical maneuvers. This is a complication that can lead to severe postoperative psychosomatic dysfunction in almost 50% of patients. (Schwender D, Kunze-Kronawitter H, Dietrich P, Klasing S, Forst H, Madler C. Conscious awareness during general anesthesia: patients' perceptions, emotions, cognition and reactions. Br J Anaesth 1998; 80:133-39). As intraoperative awareness could be a major medico-legal liability to the anesthesiologists and could lead to postoperative psychosomatic dysfunction in the patients, it should therefore be avoided. (Sandin RH, Enlund G, Samuelsson P, Lennmarken C: Awareness during anesthesia: a prospective case study. Lancet. 2000; 355:707-11. Sandin RH. Awareness 1960-2002, explicit recall of events during general anesthesia. Adv Exp Med Biol. 2003; 523:135-47).

A usual clinical method for assessing the nociception during general anesthesia is the Ramsay scale where level 6 is no response to noxious stimulation.

### Introduction to analgesia

Nociception and the perception of pain define the need for analgesia, obtaining pain relief. The state of analgesia for surgery is reached by the administration of analgesics. The demand of analgesics is individual for each patient. Therefore there is a need for continuous preferably noninvasive monitoring of the analgesia of the patient. Autonomic responses such as tachycardia, hypertension, emotional sweating and lacrimation although non-specific are regarded as signs of nociception and consequently inadequate analgesia.

A method for monitoring skin conductance have been described in US pat no. 6571124 "Apparatus and method for monitoring skin conductance and method for controlling a warning signal", however this patent is intended for use with babies and does not take a multiparameter approach as in the present patent. Additionally, the method described in this patent applies a more extended analysis of the ICG by analyzing data with a Hilbert transform.

The autonomic nerve system (ANS) consists of two branches, the parasympathetic and the sympathetic system, where a sympathetic response is activated during nociception. A US patent no 7024234 "Method and apparatus for monitoring the autonomic nervous system" describes an algorithm that analyzes a photoplethysmographic signal for the detection of ANS activity during sleep related breathing disorders.

A method for assessing the level of nociception during anesthesia has been described in US pat no. 2005143665 "Method and an apparatus for pulse plethysmograph based detection of nociception during anesthesia or sedation" by HUIKU MATTI V T (FI); KORHONEN ILKKA (FI); VAN GILS MARCUS J (FI); JOUSI MIKKO O (FI); LOTJONEN JYRKI M P (FI). This patent explores the detection of nociception by plethysmography (PM) from which a number of parameters are derived: the max amplitude of the PM curve, the amplitude of the dicrotic notch, the Heart Beat interval (RR) and other parameters from the PM curve. These parameters are used to design a final index using a multiple logistic regression approach.

In the present patent a combination of additional parameters of HR, SpO2, ICG, EEG and facial EMG is applied. This gives a safer method because the final index, termed "Composite Nociception Index" (CNI), is a combination of parameters which are independent.

In 1992 Pomfrett filed the patent application for U.S. Pat. No. 5,372,140 which proposes a method and an apparatus for providing a measure of the depth of anesthesia based on analyzing beat-to-beat heart rate together with respiration. For that purpose a series of so-called R-waves from the cardiac signal is analyzed for determining the position in time of each R-wave relative to the respiratory cycle within which it occurs, and a measurement value representing the degree of clustering is derived. Further a so-called circular statistics is utilized with a test for randomness, and finally the measurement value is compared with a reference value to find the depth of anesthesia. The proposed measure is related to respiratory sinus arrhythmia, which is primarily controlled by parasympathetic nervous system. The measure is poorly related to the functioning of the sympathetic nervous system, and hence may not measure sympathetic reactions to pain. As a conclusion, the disclosed method and apparatus does not provide results, which could be considered as an objective measure for the level of analgesia of a patient.

Cohen-laroque filed in 1998 the patent application "Device for determining the depth of anesthesia", Document U.S. Pat. No. 6,120,443. The patent describes a cardiac activity of the patient, detecting a periodic wave there from, calculating time intervals between successive waves, determining a series of time intervals, and calculating a fractal dimension of said series as well as calculating a depth of anesthesia as a function of the fractal dimension. The signal is filtered by calculating the maximum correlation between the sampled signal and a theoretical signal, whereupon a signal period between 20 and 80 intervals is suggested, then regrouping the time intervals to form several digital series, and finally the fractal dimension is approximated by determining a dimension of correlation between said digital series, whereupon two to ten series is used. The disclosed method is mathematically based on computing correlation dimension for beat-to-beat heart rate signal. Theoretically the correlation dimension requires a very long data sequence, leading to large delays in real-time monitoring. Though the inventor proposes to use relatively short data sequences to minimize the acquisition delay this makes the theoretical basis of the method questionable. As above, also this disclosed method and apparatus does not provide results, which could be considered as an objective or reliable measure for the level of analgesia of a patient.

Huiku et al filed 14/10/2002 "A method and an apparatus for pulse plethysmograph based detection of nociception during anesthesia or sedation" and in 2003 Iika Korhonen filed an application termed "Monitoring a condition of a patient during anesthesia or sedation" US pat # 6685649. Both patents refer to an invention for detection of nociception by analysis of RR intervals achieved either from the ECG or from the BP. From the RR interval the acceleration emphasized RR interval is defined. The patent is different from the present invention as it does not combine the ECG with the features extracted from EEG nor does it use the analgetics concentrations.

In another example, patent document US 5372140 recites a method and an apparatus for providing a measure of the depth of anesthesia based on analyzing beat-to-beat heart rate together with respiration, which does not provide results that could be considered as an objective measure for the level of analgesia of a patient. Patent document US 6120443 recites a method for calculating the depth of anesthesia as a function of the fractal dimension of a series of time intervals between successive waves of cardiac activity of a patient. This disclosed method is mathematically based on computing correlation dimension for beat-to-beat heart rate signal. Theoretically, the correlation dimension requires a very long data sequence, which leads to large delays in real-time monitoring.

Nociception and the perception of pain define the need for analgesia for obtaining pain relief. The state of analgesia for surgery is reached by the administration of analgesics. The demand of analgesics is individual for each patient, therefore, there is a need for continuous, preferably non-invasive, monitoring of the analgesia of the patient. Autonomic responses such as tachycardia, hypertension, emotional sweating and lacrimation, although non-specific, are regarded as signs of nociception and consequently inadequate analgesia.

Several methods for monitoring nociception were previously recited. A monitoring method by skin conductance has been claimed in patent document US 6571124, however, this method is specified for use solely with neonates and does not take a multi-parameter approach. In another example, patent document US 7024234 recites an algorithm that analyzes a photoplethysmographic signal for the detection of the autonomic nervous system activity during sleep related breathing disorders. In yet another example, patent document US 2005143665 recites a method for assessing the level of nociception during anesthesia by plethysmography from which a number of parameters are derived which are used to design a final index using a multiple logistic regression approach. In yet another example, patent document 6685649 recites a method for detection of nociception by analysis of RR intervals achieved either from ECG data or blood pressure data. From the RR intervals the acceleration emphasized RR interval is defined. Patent document US 2009/0076339 discloses a method for monitoring the nociception of a patient during general anesthesia by extracting RR intervals from ECG and blood pressure. The method is based on detection of simultaneous increase in HR and BP, defined as a non-baroreflex. However, the described method is not able to detect whether the patient has been overdosed with analgesia but rather only detect whether the patient responds or not to painful stimuli with a positive predictive value of 30%. In yet another example, patent document EP 1495715 recites a method for measuring an index of hypnosis as well as index of analgesia which are independent to each other.

Lastly, Marugg filed 10/12/2013 "a device and method for determining the probability of response to pain and nociception of a subject t". This is a method for determining the probability of response to pain and nociception (final qNOX) of a subject during different levels of arousal, comprising steps of: (a) receiving electroencephalography (EEG) data and electromyography (EMG) data; (b) defining an index of consciousness (qCON) as a function of the EEG data; (c) defining an index of nociception (initial qNOX) as a function of the EEG data and the EMG data; and, (d) defining a weighing factor alpha a as a function of qCON; wherein, if the initial qNOX > qCON and qCON < k1 a is defined by the following formula:[alpha] = k2 - k4 * (qCON - k3); where k1, k2, k3 and k4 are predetermined values; if [alpha] > k2, [alpha] is defined by the following formula [alpha] = k2; further wherein a final qNOX is defined by the following formula: final qNOX = (1 - [alpha]) * initial qNOX + [alpha]* qCON. While this publication might be considered as the closest prior art, several features of the present invention are novel, not obvious and respond to a long felt need for a better and more complete apparatus for the assessment of the level of pain and nociception during general anesthesia.

WO 2014/091291 A1 discloses a method and a device for determining the probability of response to pain and nociception comprising obtaining EEG, EMG and EGG data, performing Fast Fourier Transformations (FFT) on said data, calculating Fokker-Planck drift and diffusion coefficients between EEG and heart rate variability (HRV) data and combining the resulting information into an index of consciousness and an index of nociception.

### SUMMARY OF THE INVENTION

The invention is set out in the appended set of claims. It is one object of the invention to disclose an apparatus equipped with means for estimating pain and nociception while awake, during general anesthesia or sedation from data including electroencephalogram (EEG), facial electromyogram (EMG), heart rate variability (HRV) by electrocardiogram (ECG) and plethysmography by impedance cardiography (ICG), the apparatus comprising the following features: means for obtaining a signal containing EEG and facial EMG, said mean adapted for recording from a subjects scalp with three electrodes positioned at middle forehead, left (right) forehead and the left (right) cheek; means for obtaining a three leads ECG signal and adaptations for calculating the R-R interval and the HRV from said ECG signal; means for obtaining an ICG signal with four electrodes positioned at the chest of the patient; means for obtaining the plethysmographic from the ICG; adaptation for calculating the Fast Fourier Transform (FFT) and the Choi-Williams distributions for about 1-60 seconds of the EEG signal; adaptations for calculating the frequency spectrum in about 1-10 seconds EMG signal; adaptations for calculating the spectral edge frequency of the spectrum of the R-R interval of the ECG over about 2-6 minutes; adaptations for calculating the Hilbert transform of the ICG signal from which the number of peaks over a certain threshold in the 1st derivative of the Hilbert phase, is estimated; means adapted for combining the extracted EEG, EMG, ECG, and ICG data into a final index of nociception represented by a scale from 0 to 99, where a value of 99 represents a high probability of response to light noxious stimuli and a value of close or equal to 0 corresponds to a total block of afferent noxious stimuli, for example obtained by local anesthetic drugs.

It is hence another object of the invention to disclose the apparatus further defined as the position of the electrodes can be either middle forehead (Fp, in the international 10-20 electrode positioning system), left forehead (F7) and the left cheek (temporal process) 2 cm below the middle eye line or the electrode position can alternatively be middle forehead, right forehead and the right cheek (temporal process) 2 cm below the middle eye line.

It is hence another object of the invention to disclose the apparatus further refined by calculating the HRV by doing a Fast Fourier Transform (FFT) of the signal defined as the R-R interval; the ninety-five percent Spectral Edge Frequency (SEF) of the spectrum is used for the subsequent calculation of the Index of nociception.

It is hence another object of the invention to disclose the apparatus further refined by calculating the Hilbert transform of the plethysmographic curve.

It is hence another object of the invention to disclose the apparatus further refined by estimating the Choi-Williams distribution over about at least 1-60 s and about at least 0 - 100 Hz, the variability of the energy of 1s and 1 Hz squares are calculated.

It is hence another object of the invention to disclose the apparatus further refined by calculating the mutual information and Fokker-Planck drift and diffusion coefficients between the EEG and impedance cardiography.

It is hence another object of the invention to disclose the apparatus further defined by calculating the energy in different frequency bands of the spectrum.

It is hence another object of the invention to disclose the apparatus further defined as the principle of Fast Fourier Transform (FFT) of the R-R intervals; from the spectrum the ninety-five percent Spectral Edge Frequency (SEF) is calculated and used for the subsequent calculation of the Index of nociception.

It is hence another object of the invention to disclose the apparatus further refined as the ICG is measured with a sampling frequency of about 250 Hz whereafter the Hilbert transform is carried out on a signal of about 1 to 10 s duration; from which the number of peaks in the first derivative of the Hilbert phase which are over a certain threshold, is calculated; this parameter is used for the subsequent calculation of the Index of nociception.

It is hence another object of the invention to disclose the apparatus that describe parameters which are all included in a classifier, which could be but not necessarily a multiple linear or logistic regression, a quadratic equation or a fuzzy inference reasoner or a an adaptive neuro fuzzy inference system, where the output of the classifier is the Index of nociception, an index associated with the probability of response of the patient to noxious stimuli.

It is hence one object of the invention to disclose a method for estimating pain and for calculating the level of nociception while awake, during general anesthesia or sedation from data including electroencephalogram (EEG), facial electromyogram (EMG), heart rate variability (HRV) by electrocardiogram (ECG) and plethysmography by impedance cardiography (ICG), the method comprising the steps of: obtaining a signal recording containing EEG and facial EMG from a subjects scalp with three electrodes positioned at middle forehead, left (right) forehead and the left (right) cheek; obtaining a three leads ECG signal and calculating the R-R interval and the HRV from said ECG signal; obtaining an ICG signal with four electrodes positioned at the chest of the patient; obtaining the plethysmographic from the ICG; calculating the Fast Fourier Transform (FFT) and the Choi-Williams distributions for about 1-60 seconds of the EEG signal; calculating the frequency spectrum in a 1-10 seconds EMG signal; calculating the spectral edge frequency of the spectrum of the R-R interval of the ECG over about 2-6 minutes; calculating the Hilbert transform of the ICG signal from which the number of peaks over a certain threshold in the 1st derivative of the Hilbert phase, is estimated; combining the extracted EEG, EMG, ECG, and ICG data into a final index of nociception represented by a scale from 0 to 99, where a value of 99 represents a high probability of response to light noxious stimuli and a value of close or equal to 0 corresponds to a total block of afferent noxious stimuli, for example obtained by local anesthetic drugs.

It is hence another object of the invention to disclose the method where said electrodes can be further positioned in a place selected from group consisting of: middle forehead (Fp, in the international 10-20 electrode positioning system), left forehead (F7) and the left cheek (temporal process) 2 cm below the middle eye line or the electrode position can alternatively be middle forehead, right forehead and the right cheek (temporal process) 2 cm below the middle eye line, and any combination thereof.

It is hence another object of the invention to disclose the method where said step further comprises a step of calculating the HRV by doing a Fast Fourier Transform (FFT) of the signal defined as the R-R interval; the ninety-five percent Spectral Edge Frequency (SEF) of the spectrum is used for the subsequent calculation of the Index of nociception.

It is hence another object of the invention to disclose the method where said step further comprises a step of calculating the Hilbert transform of the plethysmographic curve.

It is hence another object of the invention to disclose the method where said step further comprises a step of estimating the Choi-Williams distribution over about at least 1-60 s and about at least 0 - 100 Hz, the variability of the energy of 1s and 1 Hz squares are calculated.

It is hence another object of the invention to disclose the method where said method further comprises a step of calculating the mutual information and Fokker-Planck drift and diffusion coefficients between the EEG and impedance cardiography.

It is hence another object of the invention to disclose the method where said step further comprises a step of calculating the energy in different frequency bands of the spectrum.

It is hence another object of the invention to disclose the method where said step is further defined as the principle of Fast Fourier Transform (FFT) of the R-R intervals; from the spectrum the ninety-five percent Spectral Edge Frequency (SEF) is calculated and used for the subsequent calculation of the Index of nociception.

It is hence another object of the invention to disclose the method where said step is further refined as the ICG is measured with a sampling frequency of about 250 Hz whereafter the Hilbert transform is carried out on a signal of 1 to 10 s duration; from which the number of peaks in the first derivative of the Hilbert phase which are over a certain threshold, is calculated; this parameter is used for the subsequent calculation of the Index of nociception.

It is hence another object of the invention to disclose the method where said method describe parameters which are all included in a classifier, which could be but not necessarily a multiple linear or logistic regression, quadratic equation or a fuzzy inference reasoner or a an adaptive neuro fuzzy inference system, where the output of the classifier is the Index of nociception, an index associated with the probability of response of the patient to noxious stimuli.

### DESCRIPTION OF THE INVENTION

The following description is provided, so as to enable any person skilled in the art to make use of the invention and sets forth the best modes contemplated by the inventor of carrying out this invention. Various modifications, however, are adapted to remain apparent to those skilled in the art, since the generic principles of the present invention have been defined specifically to provide an apparatus for the assessment of the level of pain and nociception while awake or during general anesthesia. Thus a novel method for applying such apparatus has been obtained.

The term "EEG" refers herein after to electroencephalogram.

The term "EMG" refers hereinafter to facial electromyogram.

The term "HRV" refers hereinafter to heart rate variability.

The term "ECG" refers hereinafter to electrocardiogram.

The term "ICG" refers hereinafter to plethysmography by impedance cardiography.

The term "FFT" refers hereinafter to Fast Fourier Transform.

The term "CNI" refers hereinafter to Composite Nociception Index.

The term "ANFIS" refers hereinafter to Adaptive Neuro Fuzzy Inference System.

The term "SEF" refers hereinafter to Spectral Edge Frequency.

The term "about" refers hereinafter to ±25 % of the mentioned value.

In one embodiment of the present invention the apparatus is equipped with means for measuring pain and adapted for calculating the level of nociception while awake, during general anesthesia or sedation from data including electroencephalogram (EEG), facial electromyogram (EMG), heart rate variability (HRV) by electrocardiogram (ECG) and plethysmography by impedance cardiography (ICG), the apparatus comprising the following features:
1. means for obtaining a signal containing EEG and facial EMG, said mean adapted for recording from a subjects scalp with three electrodes positioned at middle forehead, left (right) forehead and the left (right) cheek;
2. means for obtaining a three leads ECG signal and adaptations for calculating the R-R interval and the heart rate variability (HRV) from said ECG signal;
3. means for obtaining an impedance cardiography (ICG) signal with four electrodes positioned at the chest of the patient;
4. means for obtaining the plethysmographic from the impedance cardiography (ICG);
5. adaptation for calculating the Fast Fourier Transform (FFT) and the Choi-Williams distributions for 1-60 seconds of the EEG signal;
6. adaptations for calculating the frequency spectrum in a 1-10 seconds EMG signal;
7. adaptations for calculating the spectral edge frequency of the spectrum of the R-R interval of the ECG over approximately 2-6 minutes;
8. adaptations for calculating the Hilbert transform of the ICG signal from which the number of peaks over a certain threshold in the 1st derivative of the Hilbert phase, is estimated;
9. means adapted for combining the extracted EEG, EMG, ECG, and ICG data into a final index of nociception represented by a scale from 0 to 99, where a value of 99 represents a high probability of response to light noxious stimuli and a value of close or equal to 0 corresponds to a total block of afferent noxious stimuli, for example obtained by local anesthetic drugs.

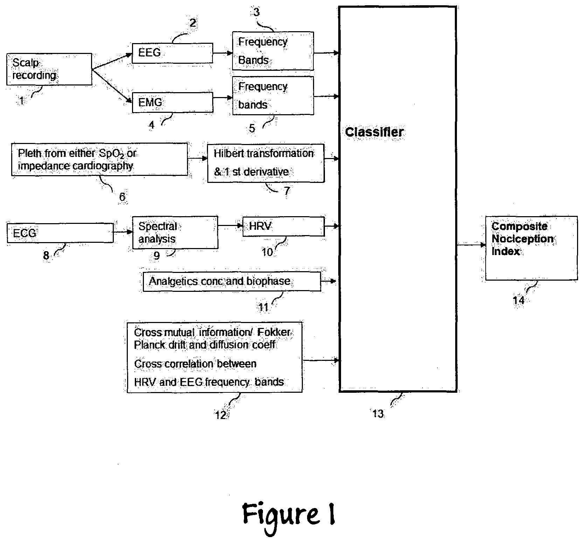

Referring now to Figure 1, showing the flow chart of the invention. The one channel EEG (2) and EMG (4) signals are recorded (1) from three surface electrodes positioned on the forehead of the subject. The ECG (8) is recorded with 4 surface electrodes positioned two on the chest and two on the neck, the Fast Fourier Transform (9) is estimated and subsequently the Heart Rate Variability is calculated (10). The parameters extracted from EEG, EMG, and HRV are all used as input to the classifier (13) which could be either a linear regression, a logistic regression, a fuzzy logic classifier, a neural network or a hybrid between a fuzzy logic system and a neural network such as an Adaptive Neuro Fuzzy Inference System (ANFIS). The output of the classifier is the Composite Nociception Index (CNI) (14). The CNI is assuming low values if the patient does not respond to noxious stimuli and high values if the patient is responding.

In one embodiment the position of the electrodes can be either middle forehead (Fp, in the international 10-20 electrode positioning system), left forehead (F7) and the left cheek (temporal process) 2 cm below the middle eye line or the electrode position can alternatively be middle forehead, right forehead and the right cheek (temporal process) 2 cm below the middle eye line.

The frequency bands (3) are extracted from the time domain of the EEG (2). An FFT is carried out of the EEG which enables the calculation of frequency ratios. The mutual information, Fokker-Planck drift and diffusion coefficients and cross correlation are calculated for the frequency bands of the EEG and ECG.

In the preferred embodiment the impedance cardiography is recorded from the same electrodes by which the ECG is recorded (6). A Hilbert transform and 1st derivative of the plethysmographic curve is used as input to the classifier (7).

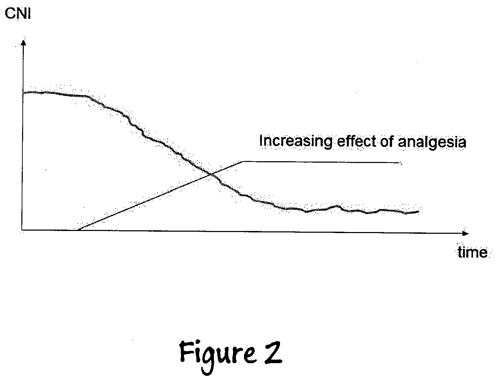

An example of the behavior of the CNI is shown in figure 2. The x-axis denotes time while the y-axis denotes CNI and the effect site concentration of an analgesics, in this example remifentanyl. When no analgesics are administered to the patient, then the CNI is high, indicating that the patient has a high probability of responding to noxious stimuli, leading to hemodynamic complications such as myocardial infarction or stroke. When the analgetics, for example remifentanil, starts to take effect, then the CNI is dropping to a lower value, indicating less probability of response to a noxious stimulus. However, a very strong noxious stimulus might cause the CNI to increase although analgesia has been administered.

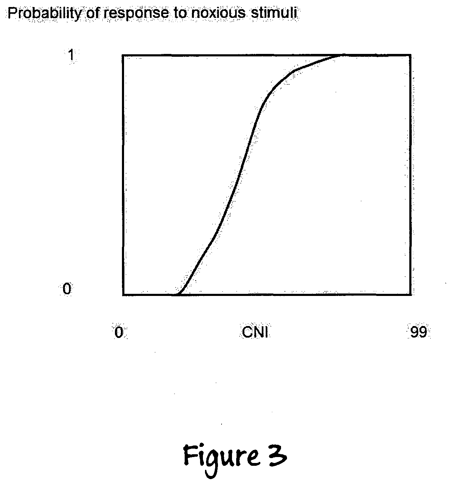

The range of the CNI could be from 0 to 99. The probability of response to noxious stimuli follows a sigmoidal shaped logistic regression. This is shown in figure 3 showing that a value of 99 of the CNI means that the probability of response to light noxious stimuli is close to 1. The definition of a CNI equal 0 is a total block of the afferent noxious stimulus.

### The Heart Rate Variability

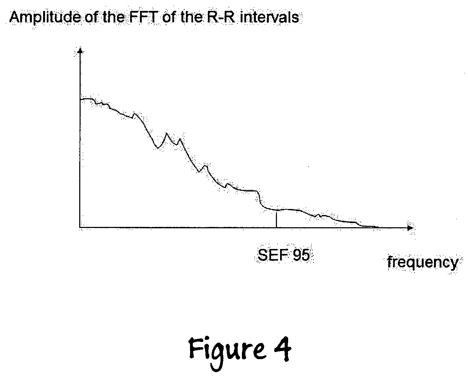

The HRV is calculated by performing a Fast Fourier Transform on the HR signal, over a 120-800 s period. From this, the Spectral Edge Frequency (SEF) is calculated as the frequency where 95 % of the area of the total spectrum is obtained, as shown in figure 4. The SEF of the HRV is used as input to the classifier as shown in figure 1 (10).

### The EEG

A Fast Fourier Transform (FFT) is applied to the EEG and the energy in frequency bands are defined. From that ratios are calculated which are used as input to the classifier. The mutual information, Fokker-Planck drift and diffusion coefficients and cross correlation are calculated for the frequencies calculated of the EEG, EMG and ECG.

### The plethysmographic curve

In the preferred embodiment, the plethysmographic curve, is constructed by injection of a current in two electrodes, while voltage between two adjacent electrodes is recorded, preferably at a frequency in the 10-200 KHz range. The impedance is then calculated and the plethysmographic curve is then defined as the impedance or the impedance cardiography.

### The analgetics concentration and biophase

The concentration of the analgetics could be, but not limited to, the plasma concentration of the infused remifentanil by an infusion pump. The biophase could be the effect site concentration of remifentanil. Either could be used as input to the model.

In a preferred embodiment of the present invention the measurements abovementioned are performed while awake, during general anesthesia or sedation.

### BRIEF DESCRIPTION OF THE FIGURES

Figure 1 showing a schematic flow chart of the present invention.
Figure 2 showing a schematic graph of the evolution of the Composite Nociception Index (CNI) during infusion of an analgesic.
Figure 3 showing a schematic graph of the sigmoidal curve showing the conceptual relationship between the Composite Nociception Index (CNI) and the probability of response to a noxious stimulus.
Figure 4 showing a schematic flow chart of the spectral analysis of the R-R intervals which is estimated by a Fast Fourier transform. The Spectral Edge Frequency (SEF) is defined as the frequency where 95% of the area or energy of the spectrum is reached.

## Claims

1. An apparatus equipped with means for estimating pain and nociception while awake, during general anesthesia or sedation from data including electroencephalogram (EEG), facial electromyogram (EMG), heart rate variability (HRV) by electrocardiogram (ECG) and plethysmography by impedance cardiography (ICG), the apparatus comprising the following features:
(a) means for obtaining a signal containing EEG and facial EMG, said mean adapted for recording from a subjects scalp with three electrodes positioned at middle forehead, left (right) forehead and the left (right) cheek;
(b) means for obtaining a three leads ECG signal and adaptations for calculating the R-R interval and the HRV from said ECG signal;
(c) means for obtaining an ICG signal with four electrodes positioned at the chest of the patient;
(d) means for obtaining the plethysmographic from the ICG;
(e) adaptation for calculating the Fast Fourier Transform (FFT) and the Choi-Williams distributions for about 1-60 seconds of the EEG signal;
(f) adaptations for calculating the frequency spectrum in about 1-10 seconds EMG signal;
(g) adaptations for calculating the spectral edge frequency of the spectrum of the R-R interval of the ECG over about 2-6 minutes;
(h) adaptations for calculating the Hilbert transform of the ICG signal from which the number of peaks over a certain threshold in the 1st derivative of the Hilbert phase, is estimated;
(i) means adapted for combining the extracted EEG, EMG, ECG, and ICG data into a final index of nociception represented by a scale from 0 to 99, where a value of 99 represents a high probability of response to light noxious stimuli and a value of close or equal to 0 corresponds to a total block of afferent noxious stimuli, for example obtained by local anesthetic drugs.

2. The apparatus according to claim 1a is further defined as the position of the electrodes can be either middle forehead (Fp, in the international 10-20 electrode positioning system), left forehead (F7) and the left cheek (temporal process) 2 cm below the middle eye line or the electrode position can alternatively be middle forehead, right forehead and the right cheek (temporal process) 2 cm below the middle eye line.

3. The apparatus according to claim 1b is further refined by calculating the HRV by doing a Fast Fourier Transform (FFT) of the signal defined as the R-R interval; the ninety-five percent Spectral Edge Frequency (SEF) of the spectrum is used for the subsequent calculation of the Index of nociception.

4. The apparatus according to claim Id is further refined by calculating the Hilbert transform of the plethysmographic curve.

5. The apparatus according to claim le is further refined by estimating the Choi-Williams distribution over about at least 1-60 s and about at least 0 - 100 Hz, the variability of the energy of 1s and 1 Hz squares are calculated.

6. The apparatus according to claim 1 which is further refined by calculating the mutual information and Fokker-Planck drift and diffusion coefficients between the EEG and impedance cardiography.

7. The apparatus according to claim 1f is further defined by calculating the energy in different frequency bands of the spectrum.

8. The apparatus according to claim 1g is further defined as the principle of Fast Fourier Transform (FFT) of the R-R intervals; from the spectrum the ninety-five percent Spectral Edge Frequency (SEF) is calculated and used for the subsequent calculation of the Index of nociception.

9. The apparatus according to claim 1h is further refined as the ICG is measured with a sampling frequency of about 250 Hz whereafter the Hilbert transform is carried out on a signal of about 1 to 10 s duration; from which the number of peaks in the first derivative of the Hilbert phase which are over a certain threshold, is calculated; this parameter is used for the subsequent calculation of the Index of nociception.

10. The apparatus according to claims 1-9 describe parameters which are all included in a classifier, which could be but not necessarily a multiple linear or logistic regression, a quadratic equation or a fuzzy inference reasoner or a an adaptive neuro fuzzy inference system, where the output of the classifier is the Index of nociception, an index associated with the probability of response of the patient to noxious stimuli.

11. A method for estimating pain and for calculating the level of nociception while awake, during general anesthesia or sedation from data including electroencephalogram (EEG), facial electromyogram (EMG), heart rate variability (HRV) by electrocardiogram (ECG) and plethysmography by impedance cardiography (ICG), the method comprising the steps of:
(a) obtaining a signal recording containing EEG and facial EMG from a subjects scalp with three electrodes positioned at middle forehead, left (right) forehead and the left (right) cheek;
(b) obtaining a three leads ECG signal and calculating the R-R interval and the HRV from said ECG signal;
(c) obtaining an ICG signal with four electrodes positioned at the chest of the patient;
(d) obtaining the plethysmographic from the ICG;
(e) calculating the Fast Fourier Transform (FFT) and the Choi-Williams distributions for about 1-60 seconds of the EEG signal;
(f) calculating the frequency spectrum in a 1-10 seconds EMG signal;
(g) calculating the spectral edge frequency of the spectrum of the R-R interval of the ECG over about 2-6 minutes;
(h) calculating the Hilbert transform of the ICG signal from which the number of peaks over a certain threshold in the 1st derivative of the Hilbert phase, is estimated;
(i) combining the extracted EEG, EMG, ECG, and ICG data into a final index of nociception represented by a scale from 0 to 99, where a value of 99 represents a high probability of response to light noxious stimuli and a value of close or equal to 0 corresponds to a total block of afferent noxious stimuli, for example obtained by local anesthetic drugs.

12. The method according to claim 11a, wherein said electrodes can be further positioned in a place selected from group consisting of: middle forehead (Fp, in the international 10-20 electrode positioning system), left forehead (F7) and the left cheek (temporal process) 2 cm below the middle eye line or the electrode position can alternatively be middle forehead, right forehead and the right cheek (temporal process) 2 cm below the middle eye line, and any combination thereof.

13. The method according to claim 11b, wherein said step further comprises a step of calculating the HRV by doing a Fast Fourier Transform (FFT) of the signal defined as the R-R interval; the ninety-five percent Spectral Edge Frequency (SEF) of the spectrum is used for the subsequent calculation of the Index of nociception.

14. The method according to claim 11d, wherein said step further comprises a step of calculating the Hilbert transform of the plethysmographic curve.

15. The method according to claim 11e, wherein said step further comprises a step of estimating the Choi-Williams distribution over about at least 1-60 s and about at least 0 - 100 Hz, the variability of the energy of 1s and 1 Hz squares are calculated.

16. The method according to claim 11, wherein said method further comprises a step of calculating the mutual information and Fokker-Planck drift and diffusion coefficients between the EEG and impedance cardiography.

17. The method according to claim 11f, wherein said step further comprises a step of calculating the energy in different frequency bands of the spectrum.

18. The method according to claim 11g, wherein said step is further defined as the principle of Fast Fourier Transform (FFT) of the R-R intervals; from the spectrum the ninety-five percent Spectral Edge Frequency (SEF) is calculated and used for the subsequent calculation of the Index of nociception.

19. The method according to claim 11h, wherein said step is further refined as the ICG is measured with a sampling frequency of about 10-1024 Hz whereafter the Hilbert transform is carried out on a signal of 1 to 10 s duration; from which the number of peaks in the first derivative of the Hilbert phase which are over a certain threshold, is calculated; this parameter is used for the subsequent calculation of the Index of nociception.

20. The method according to claims 11-19, wherein said method describe parameters which are all included in a classifier, which could be but not necessarily a multiple linear or logistic regression, quadratic equation or a fuzzy inference reasoner or a an adaptive neuro fuzzy inference system, where the output of the classifier is the Index of nociception, an index associated with the probability of response of the patient to noxious stimuli.

## Patentansprüche

1. Vorrichtung, ausgestattet mit Mitteln zum Abschätzen von Schmerzen und Nozizeption im Wachzustand, während einer Allgemeinanästhesie oder Sedierung anhand von Daten, einschließlich Elektroenzephalogramm (EEG), faziales Elektromyogramm (EMG), Herzfrequenzvariabilität (HRV) mittels Elektrokardiogramm (EKG) und Plethysmographie mittels Impedanzkardiographie (IKG), wobei die Vorrichtung die folgenden Merkmale umfasst:
(a) Mittel zum Erhalten eines EEG und faziales EMG enthaltenden Signals, wobei das Mittel zur Aufzeichnung von der Kopfhaut einer Person mit drei Elektroden, die in der Mitte der Stirn, auf der linken (rechten) Seite der Stirn und der linken (rechten) Wange platziert sind, angepasst ist;
(b) Mittel zum Erhalten eines EKG-Signals von drei Ableitungen und Adaptationen zum Berechnen des R-R-Intervalls und der HRV aus dem EKG-Signal;
(c) Mittel zum Erhalten eines IKG-Signals mit vier Elektroden, die auf der Brust des Patienten platziert sind;
(d) Mittel zum Erhalten der Plethysmographie aus dem IKG;
(e) Adaptation zum Berechnen der schnellen Fourier-Transformation (FFT) und der Choi-Williams-Verteilungen für ungefähr 1-60 Sekunden des EEG-Signals;
(f) Adaptationen zum Berechnen des Frequenzspektrums in ungefähr 1-10 Sekunden des EMG-Signals;
(g) Adaptationen zum Berechnen der spektralen Eckfrequenz des Spektrums des R-R-Intervalls des EKGs für ungefähr 2-6 Minuten;
(h) Adaptationen zum Berechnen der Hilbert-Transformation des IKG-Signals, aus der die Anzahl der Peaks über einem bestimmten Schwellenwert in der ersten Ableitung der Hilbert-Phase geschätzt wird;
(i) Mittel, die dazu angepasst sind, die extrahierten EEG-, EMG-, EKG- und IKG-Daten zu einem endgültigen Nozizeptionsindex zu kombinieren, der von einer Skala von 0 bis 99 repräsentiert wird, wobei ein Wert von 99 eine hohe Wahrscheinlichkeit für eine Reaktion auf schädliche Lichtreize repräsentiert und ein Wert von nahe bei oder gleich 0 einer Totalblockade afferenter schädlicher Reize entspricht, beispielsweise durch Lokalanästhetika.

2. Die Vorrichtung nach Anspruch 1a ist ferner dadurch definiert, dass sich die Position der Elektroden in der Mitte der Stirn (Fp, im internationalen 10-20-System zur Elektrodenplatzierung), auf der linken Seite der Stirn (F7) und auf der linken Wange (Processus temporalis) 2 cm unter der mittleren Augenlinie befinden kann, oder die Elektrodenposition kann sich alternativ in der Mitte der Stirn, auf der rechten Seite der Stirn und auf der rechten Wange (Processus temporalis) 2 cm unter der mittleren Augenlinie befinden.

3. Die Vorrichtung nach Anspruch 1b ist ferner verfeinert durch Berechnen der HRV durch Durchführung einer schnellen Fourier-Transformation (FFT) des als R-R-Intervall definierten Signals; die 95%-spektrale Eckfrequenz (SEF) des Spektrums wird für die anschließende Berechnung des Nozizeptionsindex verwendet.

4. Die Vorrichtung nach Anspruch 1d ist ferner verfeinert durch Berechnen der Hilbert-Transformation der Plethysmographiekurve.

5. Die Vorrichtung nach Anspruch 1e ist ferner verfeinert durch Schätzen der Choi-Williams-Verteilung für mindestens ungefähr 1-60 s und mindestens ungefähr 0-100 Hz, wobei die Variabilität der Energie von 1s und 1 Hz Quadrate berechnet wird.

6. Die Vorrichtung nach Anspruch 1 ist ferner verfeinert durch Berechnen der gegenseitigen Information und Fokker-Planck-Drift- und Diffusionskoeffizienten zwischen dem EEG und Impedanzkardiographie.

7. Die Vorrichtung nach Anspruch 1f ist ferner definiert durch Berechnen der Energie in verschiedenen Frequenzbanden des Spektrums.

8. Die Vorrichtung nach Anspruch 1g ist ferner definiert als Prinzip der schnellen Fourier-Transformation (FFT) der R-R-Intervalle; aus dem Spektrum wird die 95%-spektrale Eckfrequenz (SEF) berechnet und für die anschließende Berechnung des Nozizeptionsindex verwendet.

9. Die Vorrichtung nach Anspruch 1h ist ferner dadurch verfeinert, dass das IKG mit einer Abtastfrequenz von ungefähr 250 Hz gemessen wird, wonach die Hilbert-Transformation an einem Signal von ungefähr 1 bis 10 s Dauer durchgeführt wird; woraus die Anzahl der über einem bestimmten Schwellenwert liegenden Peaks in der ersten Ableitung der Hilbert-Phase berechnet wird; dieser Parameter wird für die anschließende Berechnung des Nozizeptionsindex verwendet.

10. Die Vorrichtung nach den Ansprüchen 1-9, wobei Parameter beschrieben werden, die alle in einem Klassifikator enthalten sind, bei denen es sich handeln könnte, dies aber nicht notwendigerweise der Fall sein muss, um eine multiple lineare oder logistische Regression, eine quadratische Gleichung oder Fuzzy-Inferenz-Schlussfolgerung oder ein adaptives Neuro-Fuzzy-Inferenz-System, wobei die Ausgabe des Klassifikators der Nozizeptionsindex ist, ein Index im Zusammenhang mit der Wahrscheinlichkeit einer Reaktion des Patienten auf schädliche Reize.

11. Ein Verfahren zum Abschätzen von Schmerzen und zum Berechnen des Grads der Nozizeption im Wachzustand, während einer Allgemeinanästhesie oder Sedierung anhand von Daten, einschließlich Elektroenzephalogramm (EEG), faziales Elektromyogramm (EMG), Herzfrequenzvariabilität (HRV) mittels Elektrokardiogramm (EKG) und Plethysmographie mittels Impedanzkardiographie (IKG), wobei das Verfahren die folgenden Schritte umfasst:
(a) Erhalten einer EEG und faziales EMG enthaltenden Signalaufzeichnung von der Kopfhaut einer Person mit drei Elektroden, die in der Mitte der Stirn, auf der linken (rechten) Seite der Stirn und auf der linken (rechten) Wange angeordnet sind;
(b) Erhalten eines EKG-Signals von drei Ableitungen und Berechnen des R-R-Intervalls und der HRV aus dem EKG-Signal;
(c) Erhalten eines IKG-Signals mit vier Elektroden, die auf der Brust des Patienten platziert sind;
(d) Erhalten der Plethysmographie aus dem IKG;
(e) Berechnen der schnellen Fourier Transformation (FFT) und der Choi-Williams-Verteilungen für ungefähr 1-60 Sekunden des EEG-Signals;
(f) Berechnen des Frequenzspektrums in einem 1-10-Sekunden-EMG-Signal;
(g) Berechnen der spektralen Eckfrequenz des Spektrums des R-R-Intervalls des EKGs für ungefähr 2-6 Minuten;
(h) Berechnen der Hilbert-Transformation des IKG-Signals, aus der die Anzahl der Peaks über einem bestimmten Schwellenwert in der ersten Ableitung der Hilbert-Phase geschätzt wird;
(i) Kombinieren der extrahierten EEG-, EMG-, EKG- und IKG-Daten zu einem endgültigen Nozizeptionsindex, der von einer Skala von 0 bis 99 repräsentiert wird, wobei ein Wert von 99 eine hohe Wahrscheinlichkeit für eine Reaktion auf schädliche Lichtreize repräsentiert und ein Wert von nahe bei oder gleich 0 einer Totalblockade afferenter schädlicher Reize entspricht, beispielsweise durch Lokalanästhetika.

12. Das Das Verfahren nach Anspruch 11a, wobei die Elektroden ferner an einer Stelle platziert werden können, die aus der folgenden Gruppe ausgewählt ist: Mitte der Stirn (Fp, im internationalen 10-20-System zur Elektrodenplatzierung), linke Seite der Stirn (F7) und linke Wange (Processus temporalis) 2 cm unter der mittleren Augenlinie, oder die Elektrodenposition kann sich alternativ in der Mitte der Stirn, auf der rechten Seite der Stirn und auf der rechten Wange (Processus temporalis) 2 cm unter der mittleren Augenlinie befinden, und eine beliebige Kombination davon.

13. Das Verfahren nach Anspruch 11b, wobei der Schritt ferner einen Schritt der Berechnung der HRV durch Durchführung einer schnellen Fourier-Transformation (FFT) des als R-R-Intervall definierten Signals umfasst; wobei die 95%-spektrale Eckfrequenz (SEF) des Spektrums für die anschließende Berechnung des Nozizeptionsindex verwendet wird.

14. Das Verfahren nach Anspruch 11d, wobei der Schritt ferner einen Schritt der Berechnung der Hilbert-Transformation der Plethysmographiekurve umfasst.

15. Das Verfahren nach Anspruch 11e, wobei der Schritt ferner einen Schritt der Schätzung der Choi-Williams-Verteilung für mindestens ungefähr 1-60 s und mindestens ungefähr 0-100 Hz umfasst, wobei die Variabilität der Energie von 1s und 1 Hz Quadrate berechnet wird.

16. Das Verfahren nach Anspruch 11, wobei das Verfahren ferner einen Schritt der Berechnung der gegenseitigen Information und Fokker-Planck-Drift- und Diffusionskoeffizienten zwischen dem EEG und Impedanzkardiographie umfasst.

17. Das Verfahren nach Anspruch 1f, wobei der Schritt ferner einen Schritt der Berechnung der Energie in verschiedenen Frequenzbanden des Spektrums umfasst.

18. Das Verfahren nach Anspruch 11g, wobei der Schritt ferner definiert ist als Prinzip der schnellen Fourier-Transformation (FFT) der R-R-Intervalle; aus dem Spektrum wird die 95%-spektrale Eckfrequenz (SEF) berechnet und für die anschließende Berechnung des Nozizeptionsindex verwendet.

19. Das Verfahren nach Anspruch 11h, wobei der Schritt ferner dadurch verfeinert ist, dass das IKG mit einer Abtastfrequenz von ungefähr 10-1024 Hz gemessen wird, wonach die Hilbert-Transformation an einem Signal von ungefähr 1 bis 10 s Dauer durchgeführt wird; woraus die Anzahl der über einem bestimmten Schwellenwert liegenden Peaks in der ersten Ableitung der Hilbert-Phase berechnet wird; dieser Parameter wird für die anschließende Berechnung des Nozizeptionsindex verwendet.

20. Das Verfahren nach den Ansprüchen 11-19, wobei das Verfahren Parameter beschreibt, die alle in einem Klassifikator enthalten sind, bei denen es sich handeln könnte, dies aber nicht notwendigerweise der Fall sein muss, um eine multiple lineare oder logistische Regression, eine quadratische Gleichung oder Fuzzy-Inferenz-Schlussfolgerung oder ein adaptives Neuro-Fuzzy-Inferenz-System, wobei die Ausgabe des Klassifikators der Nozizeptionsindex ist, ein Index im Zusammenhang mit der Wahrscheinlichkeit einer Reaktion des Patienten auf schädliche Reize.

## Revendications

1. Appareil équipé de moyens pour évaluer la douleur et la nociception à l'état éveillé, au cours d'une anesthésie générale ou d'une sédation, à partir de données incluant un électroencéphalogramme (EEG), un électromyogramme facial (EMG), la variabilité de la fréquence cardiaque (HRV) par électrocardiogramme (ECG) et de la pléthysmographie par cardiographie d'impédance (ICG), l'appareil comprenant les caractéristiques suivantes :
(a) un moyen pour obtenir un signal contenant un EEG et un EMG facial, ledit moyen étant prévu pour enregistrer, à partir de la surface du crâne d'un sujet avec trois électrodes positionnées au centre du front, sur la partie gauche (droite) du front et sur la joue gauche (droite) ;
(b) un moyen pour obtenir un signal d'ECG à trois conducteurs et des adaptations pour calculer l'intervalle R-R et la HRV à partir dudit signal d'ECG ;
(c) un moyen pour obtenir un signal d'ICG avec quatre électrodes positionnées sur le thorax du patient ;
(d) un moyen pour obtenir la pléthysmographie à partir de l'ICG ;
(e) adaptation pour calculer la transformation de Fourier rapide (FFT) et la distribution de Choï-Williams pendant environ 1 à 60 secondes du signal d'EEG ;
(f) des adaptations pour calculer le spectre de fréquences pendant environ 1 à 10 secondes du signal d'EMG ;
(g) des adaptations pour calculer le front de fréquence spectrale du spectre de l'intervalle R-R de l'ECG pendant environ 2 à 6 minutes ;
(h) des adaptations pour calculer la transformation de Hilbert du signal d'ICG à partir de laquelle on peut estimer le nombre de pics au-dessus d'un certain seuil dans la première dérivée de la phase de Hilbert ;
(i) des moyens prévus pour combiner les données d'EEG, EMG, ECG et ICG extraites pour créer un index final de nociception représenté par une échelle de 0 à 99, où une valeur de 99 représente une forte probabilité de réaction à de légers stimuli douloureux et une valeur proche de ou égale à 0 correspond à un blocage total des stimuli douloureux associés, par exemple obtenu par des médicaments anesthésiques locaux.

2. Appareil selon la revendication 1, point a, **caractérisé en outre en ce que** la position des électrodes peut être soit au milieu du front (Fp, dans le système international 10/20 de positionnement des électrodes), sur la partie gauche du front (F7) et sur la joue gauche (processus temporal) à 2 cm en dessous de la ligne médiane des yeux ou la position des électrodes peut, en variante, être au milieu du front, sur la partie droite du front et sur la joue droite (processus temporal) à 2 cm en dessous de la ligne médiane des yeux.

3. Appareil selon la revendication 1, point b, **caractérisé en outre par** le calcul de la HRV en effectuant une transformation de Fourier rapide (FFT) du signal défini comme l'intervalle R-R ; le front de fréquence spectrale (SEF) à quatre-vingt-quinze pour cent du spectre étant utilisé pour le calcul subséquent de l'index de nociception.

4. Appareil selon la revendication 1, point d, **caractérisé en outre par** le calcul de la transformation de Hilbert de la courbe pléthysmographique.

5. Appareil selon la revendication 1, point e, **caractérisé en outre par** l'estimation de la distribution de Choï-Williams pendant environ au moins 1 à 60 s et sur environ au moins 0 à 100 Hz, la variabilité de l'énergie de 1s et 1Hz au carré étant calculée.

6. Appareil selon la revendication 1, **caractérisé en outre par** le calcul de l'information mutuelle et de la tendance et des coefficients de diffusion de Fokker-Planck entre l'EEG et la cardiographie d'impédance.

7. Appareil selon la revendication 1, point f, **caractérisé en outre par** le calcul de l'énergie dans différentes bandes de fréquences du spectre.

8. Appareil selon la revendication 1, point g, **caractérisé en outre par** le principe de la transformation de Fourier rapide (FFT) des intervalles R-R ; à partir du spectre, le front de fréquence spectrale (SEF) à quatre-vingt-quinze pour cent étant calculé et utilisé pour le calcul subséquent de l'index de nociception.

9. Appareil selon la revendication 1, point h, **caractérisé en outre en ce que** l'ICG est mesurée avec une fréquence d'échantillonnage d'environ 250 Hz, après quoi la transformation de Hilbert est effectuée sur un signal d'une durée d'environ 1 à 10 s ; à partir de laquelle on calcule le nombre de pics dans la première dérivée de la phase de Hilbert qui sont au-dessus d'un certain seuil ; ce paramètre étant utilisé pour le calcul subséquent de l'index de nociception.

10. Appareil selon les revendications 1 à 9, décrivant des paramètres qui sont tous inclus dans un classificateur qui pourrait être, de manière non obligatoire, une régression logistique ou linéaire multiple, une équation quadratique ou un raisonneur à inférence floue ou un système d'inférence neuro-flou adaptatif, où la sortie du classificateur est l'index de nociception, un index associé à la probabilité de réaction du patient à des stimuli douloureux.

11. Procédé pour estimer la douleur et pour calculer le niveau de nociception à l'état éveillé, au cours d'une anesthésie générale ou d'une sédation, à partir de données incluant un électroencéphalogramme (EEG), un électromyogramme facial (EMG), la variabilité de la fréquence cardiaque (HRV) par électrocardiogramme (ECG) et de la pléthysmographie par cardiographie d'impédance (ICG), le procédé comprenant les étapes suivantes :
(a) l'obtention d'un enregistrement de signal contenant un EEG et un EMG facial, à partir de la surface du crâne d'un sujet avec trois électrodes positionnées au centre du front, sur la partie gauche (droite) du front et sur la joue gauche (droite) ;
(b) l'obtention d'un signal d'ECG à trois conducteurs et le calcul de l'intervalle R-R et de la HRV à partir dudit signal d'ECG ;
(c) l'obtention d'un signal d'ICG avec quatre électrodes positionnées sur le thorax du patient ;
(d) l'obtention de la pléthysmographie à partir de l'ICG ;
(e) le calcul de la transformation de Fourier rapide (FFT) et de la distribution de Choï-Williams pendant environ 1 à 60 secondes du signal d'EEG ;
(f) le calcul du spectre de fréquences pendant environ 1 à 10 secondes du signal d'EMG ;
(g) le calcul du front de fréquence spectrale du spectre de l'intervalle R-R de l'ECG pendant environ 2 à 6 minutes ;
(h) le calcul de la transformation de Hilbert du signal d'ICG à partir de laquelle on estime le nombre de pics au-dessus d'un certain seuil dans la première dérivée de la phase de Hilbert ;
(i) la combinaison des données d'EEG, EMG, ECG et ICG extraites pour créer un index final de nociception représenté par une échelle de 0 à 99, où une valeur de 99 représente une forte probabilité de réaction à de légers stimuli douloureux et une valeur proche de ou égale à 0 correspond à un blocage total des stimuli douloureux associés, par exemple obtenu par des médicaments anesthésiques locaux.

12. Procédé selon la revendication 11, étape a, dans lequel lesdites électrodes peuvent en outre être positionnées à un emplacement sélectionné parmi le groupe constitué par : le milieu du front (Fp, dans le système international 10/20 de positionnement des électrodes), la partie gauche du front (F7) et la joue gauche (processus temporal) à 2 cm en dessous de la ligne médiane des yeux ou la position des électrodes peut, en variante, être au milieu du front, sur la partie droite du front et sur la joue droite (processus temporal) à 2 cm en dessous de la ligne médiane des yeux, et toutes combinaisons de celles-ci.

13. Procédé selon la revendication 11, étape b, dans lequel ladite étape comprend en outre une étape de calcul de la HRV en effectuant une transformation de Fourier rapide (FFT) du signal défini comme l'intervalle R-R ; le front de fréquence spectrale (SEF) à quatre-vingt-quinze pour cent du spectre étant utilisé pour le calcul subséquent de l'index de nociception.

14. Procédé selon la revendication 11, étape d, dans lequel ladite étape comprend en outre une étape de calcul de la transformation de Hilbert de la courbe pléthysmographique.

15. Procédé selon la revendication 11, étape e, dans lequel ladite étape comprend en outre une étape d'estimation de la distribution de Choï-Williams pendant environ au moins 1 à 60 s et sur environ au moins 0 à 100 Hz, la variabilité de l'énergie de 1s et 1Hz au carré étant calculée.

16. Procédé selon la revendication 11, ledit procédé comprenant en outre une étape de calcul de l'information mutuelle et de la tendance et des coefficients de diffusion de Fokker-Planck entre l'EEG et la cardiographie d'impédance.

17. Procédé selon la revendication 11, étape f, dans lequel ladite étape comprend en outre une étape de calcul de l'énergie dans différentes bandes de fréquences du spectre.

18. Procédé selon la revendication 11, étape g, dans lequel ladite étape est en outre définie comme le principe de la transformation de Fourier rapide (FFT) des intervalles R-R ; à partir du spectre, le front de fréquence spectrale (SEF) à quatre-vingt-quinze pour cent étant calculé et utilisé pour le calcul subséquent de l'index de nociception.

19. Procédé selon la revendication 11, étape h, dans lequel ladite étape est en outre **caractérisée en ce que** l'ICG est mesurée avec une fréquence d'échantillonnage d'environ 10 à 1024 Hz, après quoi la transformation de Hilbert est effectuée sur un signal d'une durée de 1 à 10 s ; à partir de laquelle on calcule le nombre de pics dans la première dérivée de la phase de Hilbert qui sont au-dessus d'un certain seuil ; ce paramètre étant utilisé pour le calcul subséquent de l'index de nociception.

20. Procédé selon les revendications 11 à 19, dans laquelle ledit procédé décrit des paramètres qui sont tous inclus dans un classificateur qui pourrait être, de manière non obligatoire, une régression logistique ou linéaire multiple, une équation quadratique ou un raisonneur à inférence floue ou un système d'inférence neuro-flou adaptatif, où la sortie du classificateur est l'index de nociception, un index associé à la probabilité de réaction du patient à des stimuli douloureux.
